(19) Europäisches Patentamt
European Patent Office
Office européen des brevets

(11) **EP 3 412 769 B1**

(12) **EUROPEAN PATENT SPECIFICATION**

(45) Date of publication and mention
of the grant of the patent:
**01.09.2021 Bulletin 2021/35**

(21) Application number: **16888734.7**

(22) Date of filing: **03.02.2016**

(51) Int Cl.:
*C12N 5/078* (2010.01)    *C12N 5/073* (2010.01)
*C12N 15/10* (2006.01)    *C12M 1/42* (2006.01)
*C12Q 1/68* (2018.01)    *G01N 33/53* (2006.01)

(86) International application number:
**PCT/CN2016/073375**

(87) International publication number:
**WO 2017/132909 (10.08.2017 Gazette 2017/32)**

(54) **METHOD FOR SEPARATING TARGET CELL FROM BLOOD SAMPLE AND USE THEREOF**

VERFAHREN ZUR TRENNUNG EINER ZIELZELLE AUS EINER BLUTPROBE UND VERWENDUNG DAVON

PROCÉDÉ POUR SÉPARER UNE CELLULE CIBLE À PARTIR D'UN ÉCHANTILLON DE SANG ET UTILISATION CORRESPONDANTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**12.12.2018 Bulletin 2018/50**

(73) Proprietor: **BGI Shenzhen**
**Shenzhen, Guangdong 518083 (CN)**

(72) Inventors:
• **ZHU, Zhu**
 **Shenzhen**
 **Guangdong 518083 (CN)**
• **LIU, Ping**
 **Shenzhen**
 **Guangdong 518083 (CN)**
• **GU, Ying**
 **Shenzhen**
 **Guangdong 518083 (CN)**
• **KANG, Xiongbin**
 **Shenzhen**
 **Guangdong 518083 (CN)**
• **XIA, Jun**
 **Shenzhen**
 **Guangdong 518083 (CN)**
• **QIU, Yong**
 **Shenzhen**
 **Guangdong 518083 (CN)**

(74) Representative: **Huenges, Martin**
**Maiwald Patentanwalts- und**
**Rechtsanwaltsgesellschaft mbH**
**Elisenhof**
**Elisenstraße 3**
**80335 München (DE)**

(56) References cited:
**WO-A1-2015/175562    WO-A2-02/40540**
**WO-A2-2007/147076    CN-A- 102 690 786**
**US-A1- 2014 228 226**

• **AKIHIKO SEKIZAWA ET AL: "Recent advances in non-invasive prenatal DNA diagnosis through analysis of maternal blood", JOURNAL OF OBSTETRICS AND GYNAECOLOGY RESEARCH, vol. 33, no. 6, 12 November 2007 (2007-11-12), pages 747-764, XP055608925, JP ISSN: 1341-8076, DOI: 10.1111/j.1447-0756.2007.00652.x**
• **GUO FAN ET AL: "The Transcriptome and DNA Methylome Landscapes of Human Primordial Germ Cells", CELL, ELSEVIER, AMSTERDAM, NL, vol. 161, no. 6, 4 June 2015 (2015-06-04), pages 1437-1452, XP029171296, ISSN: 0092-8674, DOI: 10.1016/J.CELL.2015.05.015**
• **WANG, XINGTONG et al.: "Recent Advances in the Application of Fetal Cells in Maternal Blood to Non-Invasive Prenatal Diagnosis", CHINESE JOURNAL OF BIRTH HEALTH & HEREDITY, vol. 17, no. 9, 25 September 2009 (2009-09-25), pages 5-7,17, XP009511568, ISSN: 1006-9534**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- SEKIZAWA, A. et al.: "Recent Advances in Non-Invasive Prenatal DNA Diagnosis through Analysis of Maternal Blood", JOURNAL OF OBSTETRICS AND GYNAECOLOGY RESEARCH, vol. 33, no. 6 12 November 2007 (2007-11-12), pages 747-764, XP008144742, ISSN: 1341-8076 Retrieved from the Internet: URL:https://doi.org/10.1111/j.1447-0756.2007.00652.x

- "Quality Scores for Next-Generation Sequencing - Assessing sequencing accuracy using Phred quality scoring", Illumina, Inc. , 31 October 2011 (2011-10-31), Retrieved from the Internet: URL:https://www.illumina.com/documents/products/technotes/technote_Q-Scores.pdf [retrieved on 2021-02-18]

**Description**

**CROSS-REFERENCE TO RELATED APPLICATION**

None

**FIELD**

The present disclosure relates to the field of biotechnology, more particular to a method for isolating a target cell from a blood sample and use thereof.

**BACKGROUND**

Recently, with the development of high-throughput sequencing technology, it has been widely recognized to determine fetal chromosome-based copy number variation by detecting cell-free fetal DNAs in peripheral blood of a pregnant woman with a high-throughput sequencing method. However, use of the free fetal DNAs has inherent deficiencies. For example, in addition to the cell-free fetal DNAs, a large amount of maternal DNAs is also present in maternal blood. Furthermore, the cell-free fetal DNAs are found as fragmented sequences, i.e., incomplete genome. In view of the above, the existing detection mainly directs to chromosome-base copy number variation (CNV), there exist great difficulties for detecting gene mutation at a specific locus.
WO2015/175562 relates to isolating single fetal cells from a maternal blood sample. US 2014/228226 relates to determining aneuploidy in a single cell.
Therefore, there is still a need to improve detection of cell-free fetal DNAs in peripheral blood of a pregnant woman.

**SUMMARY**

The subject matter of the invention is as defined in the appended claims.
Embodiments of the present disclosure aim at to solve at least one of the problems existing in the related art, or to provide at least a commercial choice. For this purpose, in one aspect, the invention provides a method for isolating a target cell from a blood sample for analysis of the whole genome of the target cell, wherein the target cell is a fetal cell in form of a single cell, said method comprises steps of:

(1) subjecting the blood sample to immunomagnetic bead sorting, thereby obtaining a first sorted product containing the target cell,
wherein the immunomagnetic bead sorting uses beads coated with an antibody,
wherein the antibody bound on the bead is specific for a surface antigen expressed exclusively on non-target cells, and
wherein the target cell comprised in the first sorted product is not bound to the antibody used for the immunomagnetic bead sorting;
(2) staining a surface antigen on the first sorted product, followed by incubation, centrifugation and washing, thereby obtaining a precipitate containing the target cell, said surface antigen being at least one selected from the group consisting of CD45, CD14, CD56, CD19 and CD20; suspending the resulting precipitate with a buffer, thereby obtaining a first solution containing the target cell; incubating the first solution containing the target cell with Hoechst 33342/Propidium Iodide (PI) dyes at room temperature in a dark place, thereby obtaining a second solution containing stained cells; and sorting the second solution containing stained cells which is diluted in advance by flow cytometer, collecting as a second sorted product cells which are Hoechst-positive, PI negative, and negative for said at least one surface antigen used in the surface antigen staining of step (2); and
(3) separating the second sorted product by mouth-controlled pipette, thereby obtaining the target cell in form of the single cell.

In another aspect, the invention provides a device for isolating a target cell from a blood sample for analysis of the whole genome of the target cell, wherein the target cell is a fetal cell in form of a single cell, said device comprises:

an immunomagnetic bead sorting unit, configured to subject the blood sample to immunomagnetic bead sorting by using beads coated with an antibody specific for a surface antigen expressed exclusively on non-target cells, so as to obtain a first sorted product containing the target cell, wherein the target cell comprised in the first sorted product is not bound to the antibody used for the immunomagnetic bead sorting;
a flow cytometry sorting unit, connected to the immunomagnetic bead sorting unit and configured to perform the step (2) as defined the method of the invention; and
a mouth-controlled pipette isolating unit, connected to the flow cytometry sorting unit and configured to separate the second sorted product by mouth-controlled pipette, so as to obtain the target cell in form of the single cell.

It should be noted that the present disclosure is accomplished by present inventors based on the following findings.

Although it has been reported in papers and patents that fetal cells have been successfully isolated from peripheral blood of a pregnant woman, such isolated fetal cells are mainly useful in certain prenatal diagnosis, e.g., sex determination or chromosome-based copy number variation diagnosis (such as, Trisomy 21, Trisomy 13, Trisomy 18 and the like) by means of PCR or fluorescence in situ hybridization (FISH), rather than detection of gene mutation at a specific locus (such as a monogenic disease) because of the incomplete genome.

It is found by the present inventors through massive investigations that it is impossible for the existing methods to obtain a fetal cell with a complete genome for the reasons that (1) lack of an antibody with high specificity, leading to low accuracy and poor purity for acquisition of the fetal cell (i.e., a target cell); (2) inherent vulnerability of the fetal cell, leading to the fact that it is impossible for the target cell (even in high purity obtained by combined isolating methods) to contain the complete genome because the target cell is easy to be damaged by the isolating methods. In the latter regard, more isolating steps give rise to higher probability of damage, thereby further increasing the difficulty in obtaining the fetal cell with the complete genome. Nevertheless, there is none research report showing successful isolation of the fetal cell with the complete genome from maternal blood, for whole genome sequencing in high depth.

With a series of researches, the present inventors provide a means for isolating a target cell with a complete genome from a blood sample effectively, which is unexpectedly useful in detection and analysis of the fetal whole genome of the peripheral blood of the pregnant woman.

In one aspect of the present disclosure, provided in embodiments is a method for isolating a target cell in form of fetal cell in form of a single cell from a blood sample.

With this method described in this aspect, the target cell with the complete genome can be isolated and acquired effectively from the blood sample and the target cell obtained is in form of the single cell. It is surprisingly discovered by the present inventors that the single cell obtained (i.e., the target cell with the complete whole genome) is suitable for whole genome amplification, with a whole genome amplified product useful in library construction and sequencing, thus benefiting acquisition of the whole genome sequence information of the target cell in an accurate and effective way (for example, by constructing a sequencing library and whole genome sequencing). Further, based on such whole genome sequence information of the target cell, it is possible to accurately determine fetal physical condition, such as gender, genetic relationship, chromosomal abnormality, single nucleotide polymorphism, microdeletion, microrepeats and the like. That is to say, the single cell obtained by the method according to embodiments is useful in detection and analysis of the whole genome of the fetal cell in the peripheral blood of the pregnant woman.

To be noted, the term "a complete genome" in the expression "a target cell with a complete genome" used herein means the genome is complete in a relative than absolute sense. Specifically, in the related art, a target cell can be considered to be of a complete genome in the case that: after whole genome amplification and high-depth sequencing for the target cell isolated, whole genome sequencing data with high quality is obtainable for further high-depth sequencing analysis and thus effective detection for gene mutation at a specific locus.

In some embodiments of the present disclosure, the blood sample is from a pregnant woman.

In some embodiments of the present disclosure, prior to step (1), the method further includes removing red blood cells from the blood sample.

In some embodiments of the present disclosure, the blood sample is contacted with a red blood cell lysis buffer in the presence of an anticoagulant, so as to remove the red blood cells from the blood sample.

In some embodiments of the present disclosure, the anticoagulant is at least one selected from the group consisting of heparin, ethylenediamine tetraacetic acid, citrate, oxalate and ACD anticoagulant.

In some embodiments of the present disclosure, removing the red blood cells from the blood sample further includes: mixing the blood sample with a BD lysing solution diluted in advance till to solution clarification, followed by centrifugation and collection of the remaining precipitate after supernatant removal; and repeatedly washing the precipitate with a buffer containing fetal bovine serum (FBS) to remove residual BD lysing solution thoroughly. The term "repeatedly washing" in this context means the precipitate is washed with a buffer at least twice, such as twice, three times, four times, five times, six times or more.

In some embodiments of the present disclosure, a bead coated with an antibody is used for the immunomagnetic bead sorting; preferably, the antibody is a negative antibody non-specific for the target cell.

In some embodiments of the present disclosure, the antibody is against at least one of antigens selected from CD45, CD14, CD56, CD19 and CD20.

In some embodiments of the present disclosure, step (1) further includes: mixing the blood sample and beads coated with the antibody, incubating and centrifuging in sequence, followed by collection of a first precipitate after supernatant removal; suspending the first precipitate with a buffer, thereby obtaining a solution containing the target cell; loading the solution containing the target cell onto a sorting column rinsed in advance, followed by collection of entire outflow as first outflow containing the target cell; rinsing the sorting column twice with the buffer and respectively collecting respective entire outflows as second outflow and third outflow, both containing the target cell; combining the first outflow, the second outflow and the third outflow, followed by centrifugation and collection of a second precipitate; and suspending the second precipitate with the buffer, thereby obtaining the first sorted product containing the target cell.

In some specific embodiments of the present disclosure, the step (2) further includes: staining a surface antigen on the first sorted product with the antibody in a ratio of 1:50, followed by incubation, centrifugation and washing, thereby obtaining a precipitate containing the target cell, said surface antigen being at least one selected from the group consisting of CD45, CD14, CD56, CD19 and CD20; suspending the resulting precipitate with a buffer, thereby obtaining a first solution containing the target cell; incubating the first solution containing the target cell with Hoechst 33342/Propidium Iodide (PI) dyes at room temperature in a dark place, thereby obtaining a second solution containing stained cells; and sorting the second solution containing stained cells which is diluted in advance by flow cytometer, with Hoechst-positive, PI and CD45-negative cells collected as the second sorted product.

In some embodiments of the present disclosure, step (3) further includes: placing the second sorted product onto a slide, and picking up a single cell having complete cell morphology under a bright field and exhibiting desired staining results under a fluorescence field by use of dyes.

In some specific embodiments of the present disclosure, the step (3) further includes: placing the second sorted product onto a slide, and picking up a nucleated single cell having complete cell morphology under a bright field and exhibiting desired staining results under a fluorescence field into individual wells with a base solution of a plate, thereby obtaining the target cell after centrifuging.

In one aspect, the invention provides a device for isolating a target cell from a blood sample for analysis of the whole genome of the target cell, wherein the target cell is a fetal cell in form of a single cell, said device comprises:

an immunomagnetic bead sorting unit, configured to subject the blood sample to immunomagnetic bead sorting by using beads coated with an antibody specific for a surface antigen expressed exclusively on non-target cells, so as to obtain a first sorted product containing the target cell, wherein the target cell comprised in the first sorted product is not bound to the antibody used for the immunomagnetic bead sorting;
a flow cytometry sorting unit, connected to the immunomagnetic bead sorting unit and configured to perform the step (2) as according to the method of the invention; and
a mouth-controlled pipette isolating unit, connected to the flow cytometry sorting unit and configured to separate the second sorted product by mouth-controlled pipette, so as to obtain the target cell in form of the single cell.

With the device which is useful in implementing the method described above, the target cell with the complete genome can be isolated and acquired effectively from the blood sample, and targeted cell obtained is in form of the single cell, which is particularly suitable for whole genome amplification, with a whole genome amplified product useful in library construction and sequencing, thus benefiting acquisition of the whole genome sequence information of the target cell in an accurate and effective way (for example, by constructing a sequencing library and whole genome sequencing). Further, based on such whole genome sequence information of the target cell, it is possible to accurately determine fetal physical condition, such as gender, genetic relationship, chromosomal abnormality, single nucleotide polymorphism, microdeletion, microrepeats and the like. That is to say, the single cell obtained by the device according to embodiments is useful in detection and analysis of the whole genome of the fetal cell in the peripheral blood of the pregnant woman.

In some embodiments of the present disclosure, the blood sample is from a pregnant woman.

In some embodiments of the present disclosure, the device further includes a red blood cell removing unit, configured to remove red blood cells from the blood sample.

In a specific embodiment of the present disclosure, the red blood cell removing unit is configured to remove red blood cells from the blood sample by: contacting the blood sample with a red blood cell lysis buffer in the presence of an anticoagulant, so as to remove the red blood cells from the blood sample.

In some embodiments of the present disclosure, the anticoagulant is at least one selected from the group consisting of heparin, ethylenediamine tetraacetic acid, citrate, oxalate and ACD anticoagulant.

In a specific embodiment of the present disclosure, the red blood cell removing unit is configured to remove red blood cells from the blood sample by: mixing the blood sample with a BD lysing solution diluted in advance till to solution clarification, followed by centrifugation and collection of the remaining precipitate after supernatant removal; and repeatedly washing the precipitate with a buffer containing fetal bovine serum (FBS) to remove residual BD lysing solution thoroughly. The term "repeatedly washing" in this context means the precipitate is washed with a buffer at least twice, such as twice, three times, four times, five times, six times or more.

In some embodiments of the present disclosure, a bead coated with an antibody is used for the immunomagnetic bead sorting unit; preferably, the antibody is a negative antibody non-specific for the target cell.

In some embodiments of the present disclosure, the antibody is against at least one of antigens selected from CD45, CD14, CD56, CD19 and CD20.

In some embodiments of the present disclosure, the immunomagnetic bead sorting unit is configured to subject the blood sample to immunomagnetic bead sorting by: mixing the blood sample and beads coated with the antibody, incubating and centrifuging in sequence, followed by collection of a first precipitate after supernatant removal; suspending the first precipitate with a buffer, thereby obtaining a solution containing the target cell; loading the solution containing the target cell onto a sorting column rinsed in advance, followed by collection of entire outflow as first outflow containing the target cell; rinsing the sorting column twice with the buffer and respectively collecting respective entire outflows as second outflow and third outflow, both containing the target cell; combining the first outflow, the second outflow and the third outflow, followed by centrifugation and collection of a second precipitate; and to suspend the second precipitate with the buffer, thereby obtaining the first sorted product containing the target cell.

In some embodiments of the present disclosure, the flow cytometry sorting unit is configured to subject the first sorted product to flow cytometry sorting by: staining a surface antigen on the first sorted product with the antibody, said surface antigen being at least one selected from the group consisting of CD45, CD14, CD56, CD19 and CD20.

In a specific embodiment of the present disclosure, the flow cytometry sorting unit is configured to subject the first sorted product to flow cytometry sorting by: staining a surface antigen on the first sorted product with the antibody in a ratio of 1:50, followed by incubation, centrifugation and washing, thereby obtaining a precipitate containing the target cell, said surface antigen being at least one selected from the group consisting of CD45, CD14, CD56, CD19 and CD20; suspending the resulting precipitate with a buffer, thereby obtaining a first solution containing the target cell; incubating the first solution containing the target cell with Hoechst 33342/Propidium Iodide (PI) dyes at room temperature in a dark place, thereby obtaining a second solution containing stained cells; and sorting the second solution containing stained cells which is diluted in advance by flow cytometer, with Hoechst-positive, PI and CD45-negative cells collected as the second sorted product.

In some embodiments of the present disclosure, the mouth-controlled pipette isolating unit is configured to separate the second sorted product by mouth-controlled pipette by: placing the second sorted product onto a slide, and picking up a single cell having complete cell morphology under a bright field and exhibiting desired staining results under a fluorescence field by use of dyes.

In a specific embodiment of the present disclosure, the mouth-controlled pipette isolating unit is configured to separate the second sorted product by mouth-controlled pipette by: placing the second sorted product onto a slide, and picking up a nucleated single cell having complete cell morphology under a bright field and exhibiting desired staining results under a fluorescence field into individual wells with a base solution of a plate, thereby obtaining the target cell after centrifuging.

In a further aspect of the present disclosure, provided in embodiments is a method for subjecting a target cell in a blood sample to whole genome amplification, including: isolating the target cell from the blood sample by the method described above, thereby obtaining the target cell in form of a single cell; lysing the target cell, thereby obtaining a lysed solution containing the whole genome of the target cell; and amplifying the whole genome of the target cell by using a polymerase.

In some embodiments of the present disclosure, the polymerase is Phi29 DNA polymerase.

In a specific embodiment of the present disclosure, amplifying the whole genome of the target cell is conducted in an amplification system, including $10\times$Phi29 buffer, water, 1.8 to $2.2\times10^{-8}$mol deoxyribonucleotides (dNTPs), N8 primers (at a total concentration of 1.8 to $2.2\times10^{-11}$mol), 4.5 to $5.5\times10^{-3}$mg BSA, 0.0045 to 0.0055$\mu$L Pluronic F68 and 9 to 11U Phi29 DNA polymerase, wherein the N8 primers are random primers consisting of 8 dNTPs arranged randomly (i.e., Adenosine deoxytriphosphate (dATP), Thymine deoxytriphosphate (dTTP), Cytosine deoxytriphosphate (dCTP) and Guanosine deoxytriphosphate (dGTP)).

In some embodiments of the present disclosure, the method for subjecting a target cell in a blood sample to whole genome amplification further includes: (1) isolating the target cell from the blood sample by the method as described above, thereby obtaining the target cell in form of a single cell; (2) incubating the target cell with 3.7 $\mu$L PBS buffer and 3 $\mu$L lysing buffer for cell denaturation at 65°C for 10 minutes, followed by addition of 3 $\mu$L neutralizing buffer, thereby obtaining a solution as a template in a volume of 9.7 $\mu$L; and (3) incubating the solution as a template in an amplification system (for one-tube reaction) at 30°C for 3 hours, followed by incubation again at 65°C for 5 minutes, thereby obtaining a whole genome amplified product.

The amplification system (40.3 $\mu$L in total for one-tube reaction) consists of 30 $\mu$L $H_2O$, 5 $\mu$L $10\times$Phi29 buffer, 2 $\mu$L dNTPs, 2 $\mu$L N8 primers, 0.25 $\mu$L 20 mg/mL BSA (aqueous), 0.05$\mu$L 10% Pluronic F68 and 1 $\mu$L Phi29 DNA polymerase.

dNTPs include dATP, dTTP, dCTP and dGTP (each in a concentration of 10 mM).

The N8 primers are at a total concentration of 10 $\mu$M.

For the 10% Pluronic F68, 10% represents a ratio of concentration to volume.

The Phi29 DNA polymerase is at a concentration of 10 U/μL.

The N8 primers are random primers consisting of 8 dNTPs (i.e., dATP, dTTP, dCTP and dGTP) arranged randomly.

The PBS buffer in a specific embodiment is of a concentration of 0.1 M and a pH value of 7.5.

The lysing buffer for cell denaturation consists of a solute and a solvent. The solvent is water. The solutes and concentrations thereof are shown as below: 0.09 to 0.11 mM KOH, 0.9 to 1.1 mM EDTA, and 0.09 to 0.11 M DTT.

The neutralizing buffer consists of a solute and a solvent. The solvent is water. The solutes and concentrations thereof are shown as below: 54 to 66 mM $KH_2PO_4$ and 4.5 to 5.5 mM $K_2HPO_4$.

The lysing buffer for cell denaturation consists of a solute and a solvent. The solvent is water. The solutes and specific concentrations thereof are shown as below: 0.1 mM KOH, 1 mM EDTA and 0.1 M DTT.

The neutralizing buffer consists of a solute and a solvent. The solvent is water. The solutes and specific concentrations thereof are shown as below: 60 mM $KH_2PO_4$ and 5mM $K_2HPO_4$.

Thus, the whole genome amplified (WGA) product for the single cell achieves high coverage across the whole genome and excellent homogeneity, which is suitable for identifying gene identity based on short tandem repeat (STR) genotyping and analyzing the chromosome-based copy number variation in the genome, thus benefiting the detection and analysis of the whole genome of the fetal cell in the peripheral blood of the pregnant woman.

With this method described in this aspect, the target cell with the complete genome can be isolated effectively from a blood sample, and thus it is possible to achieve the whole genome amplification for the target cell, with a whole genome amplified product useful in library construction and sequencing, thus benefiting acquisition of the whole genome sequence information of the target cell in an accurate and effective way (for example, by constructing a sequencing library and whole genome sequencing). Further, based on such whole genome sequence information of the target cell, it is also possible to accurately determine fetal physical condition, such as gender, genetic relationship, chromosomal abnormality, single nucleotide polymorphism, microdeletion, microrepeats and the like. That is to say, the single cell obtained by the method according to embodiments is useful in detection and analysis of the whole genome of the fetal cell in the peripheral blood of the pregnant woman.

In a still further aspect of the present disclosure, provided in embodiments is a method for determining cell species of a target cell in a blood sample. In some embodiments of the present disclosure, the method includes: subjecting the target cell in the blood sample to whole genome amplification by the method described above, thereby obtaining a whole genome amplified product; and identifying the cell species of the target cell based on the whole genome amplified product.

It is found by the present inventors that, with this method described in the present aspect, the cell species of the target cell in the blood sample can be determined in an effective and reproducible manner with an accurate and reliable result, thus benefiting the detection and analysis of the whole genome of the fetal cell in the peripheral blood of the pregnant woman effectively.

In some embodiments of the present disclosure, the cell species of the target cell may be identified by subjecting the whole genome amplified product to Q-PCR or STR analysis.

In a still further aspect of the present disclosure, provided in embodiments is an apparatus for subjecting a target cell in a blood sample to whole genome amplification. In some embodiments of the present disclosure, the apparatus includes: a target cell isolating device, which is the device for isolating a target cell from a blood sample described above; a lysing device, connected to the target cell isolating device and configured to lyse the target cell, so as to obtain a lysed solution containing the whole genome of the target cell; and a whole genome amplification device, connected to the lysing device and configured to amplify the whole genome of the target cell using a polymerase.

With the apparatus which is useful in implementing the method for subjecting a target cell in a blood sample to whole genome amplification described above, the target cell with the complete genome can be isolated effectively from the blood sample, and thus it is possible to achieve the whole genome amplification for the target cell, with a whole genome amplified product useful in library construction and sequencing, thus benefiting acquisition of the whole genome sequence information of the target cell in an accurate and effective way (for example, by constructing a sequencing library and whole genome sequencing). Further, based on such whole genome sequence information of the target cell, it is also possible to accurately determine fetal physical condition, such as gender, genetic relationship, chromosomal abnormality, single nucleotide polymorphism, microdeletion, microrepeats and the like. That is to say, the single cell obtained by the apparatus according to embodiments is useful in detection and analysis of the whole genome of the fetal cell in the peripheral blood of the pregnant woman.

In a still further aspect of the present disclosure, provided in embodiments is a system for determining cell species of a target cell in a blood sample. In some embodiments of the present disclosure, the system include: the apparatus for subjecting the target cell in a blood sample to whole genome amplification as described above, configured to subject the target cell in the blood sample to whole genome amplification, so as to obtain a whole genome amplified product; and a cell species identifying device, connected to the whole genome amplification device as described above and configured to identify the cell species of the target cell based on the whole genome amplified product.

It is found by the present inventors that, with this system in this aspect, the cell species of the target cell in the blood sample can be determined in an effective and reproducible manner with an accurate and reliable result, thus benefiting the detection and analysis of the whole genome of the fetal cell in the peripheral blood of the pregnant woman effectively.

In some embodiments of the present disclosure, the cell species identifying device is configured to identify the cell species of the target cell by subjecting the whole genome amplified product to Q-PCR or STR analysis.

In a still further aspect of the present disclosure, provided in embodiments is a method of constructing a whole genome sequencing library of a target cell in a blood sample. In some embodiments of the present disclosure, the method includes: subjecting the target cell in the blood sample to whole genome amplification by the method described above, thereby obtaining a whole genome amplified product; and constructing a whole genome sequencing library based on the whole genome amplified product.

With this method described in this aspect, the target cell with the complete genome can be isolated effectively from the blood sample, and thus it is possible to achieve construction of the whole genome sequencing library for the target cell for convenience of whole genome sequencing, thus obtaining the whole genome sequence information of the target cell. Further, based on such the whole genome sequence information of the target cell, it is also possible to accurately determine fetal physical condition, such as gender, genetic relationship, chromosomal abnormality, single nucleotide polymorphism, microdeletion, microrepeats and the like. That is to say, the single cell obtained by the method according to embodiments is useful in detection and analysis of the whole genome of the fetal cell in the peripheral blood of the pregnant woman.

In a still further aspect of the present disclosure, provided in embodiments is a method for subjecting a target cell in a blood sample to whole genome sequencing. In embodiments of the present disclosure, the method includes: constructing a whole genome sequencing library by the method described above; and subjecting the whole genome sequencing library to sequencing.

With this method described in this aspect, the whole genome of the target cell obtained from the blood sample can be subjected to whole genome sequencing effectively, thereby obtaining the whole genome sequence information of the target cell (i.e. a single cell), thus based on such sequence information of the whole genome of the target cell, it is also possible to accurately determine fetal physical condition, such as gender, genetic relationship, chromosomal abnormality, single nucleotide polymorphism, microdeletion, microrepeats and the like. That is to say, the single cell obtained by the method according to embodiments is useful in detection and analysis of the whole genome of the fetal cell in the peripheral blood of the pregnant woman.

In some embodiments of the present disclosure, the whole genome sequencing is perfomed on at least one of sequencing platforms selected from the group consisting of Illumina-Solexa, ABI-Solid, Roche-454 and a single molecule sequencer.

In a still further aspect of the present disclosure, provided in embodiments is a method for determining a whole genome sequence information of a target cell in a blood sample. In embodiments of the present disclosure, the method includes: subjecting the target cell to whole genome sequencing by the method described above, thereby obtaining a sequencing result, and determining the whole genome sequence information of the target cell based on the sequencing result.

With this method described in this aspect, the target cell with the complete genome can be isolated from a blood sample effectively, and thus it is possible to achieve determination of the whole genome sequence information of the target cell. Further, based on such whole genome sequence information of the target cell, it is also possible to accurately determine fetal physical condition, such as gender, genetic relationship, chromosomal abnormality, single nucleotide polymorphism, microdeletion, microrepeats and the like. That is to say, the single cell obtained by the method according to embodiments is useful in detection and analysis of the whole genome of the fetal cell in the peripheral blood of the pregnant woman.

In some embodiments of the present disclosure, the blood sample is from a pregnant woman, and the target cell is a single cell derived from a fetus.

In some embodiments of the present disclosure, the method further includes: determining fetal physical condition based on the sequence information of the target cell.

In some embodiments of the present disclosure, the fetal physical condition includes gender, genetic relationship, chromosomal abnormality, single nucleotide polymorphism, microdeletion and microrepeats.

According to embodiments of the present disclosure, at least one of advantages can be achieved as follows.

1. In terms of an antibody marker, a negative antibody is exclusively used as the marker in the present disclosure, which is bound to a surface antigen expressed exclusively on non-fetal cells rather than fetal cells (i.e. fetal cells do not express the surface antigen bound to the negative antibody, e.g., an antibody specific for CD45 expressed on leukocyte), thereby overcoming disadvantages of the non-specificity of a positive antibody for fetal cells, and providing advantages of not aiming at particular fetal cell type such that the fetal cells can be isolated as many as possible, including rare fetal cells.

2. In terms of isolating methods, the present disclosure combines advantages of rapidness and effectiveness of immunomagnetic bead sorting with high purity of flow cytometry sorting, as well as single cell separation by mouth-controlled pipette, thus it is achievable for detection of fetal heritable variation in conjunction with whole genome amplification for a single cell, high-throughput sequencing and bioinformation analysis in clinical diagnosis.

3. In terms of clinical diagnosis, a fetal cell can be isolated noninvasively by the present method, thereby preventing a pregnant woman under detection from unintentional injure (such as abortion) caused by invasive technique (such as extraction of amniotic fluids).

4. In embodiments of the present disclosure, the fetal cell obtained contains a complete genome, which is particular useful in detection of gene mutation at a specific locus, especially for a fetal monogenic disease.

5. To be noted, it is impossible for the existing methods to obtain the fetal cell with the complete genome; and it is extremely difficult for combined isolating methods to isolate an entire single cell on account of damage caused by each manipulating step, i.e., more isolating steps give rise to higher probability of damage, thereby further increasing the difficulty in obtaining the target cell with the complete genome. However, according to embodiments of the present disclosure, the target cell with the complete genome is isolated effectively for high-depth sequencing analysis. Such a finding, obtained through massive experiments by the present inventors, achieves a surprising and unexpected effect, which is contrary to the common knowledge described previously, i.e., "more isolating steps give rise to higher probability of damage, thereby further increasing the difficulty in obtaining the target cell with the complete genome".

## DETAILED DESCRIPTION

The embodiments of the present disclosure are described in detail below. Such embodiments are explanatory, and aim at to explain the present disclosure rather than to limit the present disclosure.

Terms such as "first" and "second" are used herein for purposes of description and are not intended to indicate or imply relative importance or significance or impliedly indicate quantity of the technical feature referred to. Thus, the feature defined with "first" and "second" may comprise one or more this feature. In the description of the present disclosure, "a plurality of' means two or more than two this features, unless specified otherwise.

Reference will be made in detail to examples of the present disclosure. It would be appreciated by those skilled in the art that the following examples are explanatory, and cannot be construed to limit the scope of the present disclosure. The techniques specified in the examples are well known to those skilled in the art, and can be performed according to conventional methods in the art (e.g., Molecular Cloning: A Laboratory Manual, 3rd Ed.) or according to the product instructions. In addition, the reagents or instruments used in the present disclosure may be commercially available, as well the sources, trade names and necessary components of the reagents should be indicated when described at first time.

**Example 1 Pretreatment of** peripheral **blood sample and sorting target cells**

In the example, the starting blood sample is 10 mL peripheral blood sample derived from a healthy pregnant woman carrying a male fetus in 13-week gestation period, with the sample label of CFCCYY.

The 10 mL peripheral blood sample collected in a blood collection tube with heparin was mixed with 100 mL BD lysing solution (IX) diluted in advance at room temperature under gentle stirring, till to solution clarification, followed by centrifugation and collection of the remaining precipitate after supernatant removal. The precipitate obtained was washed twice with a PBS buffer containing 2% FBS, and then suspended into 80 $\mu$L of the PBS buffer containing 2% FBS, by blowing and suction to be uniform, thus obtaining a first solution without red blood cells.

To the first solution obtained, beads coated with CD45 antibody were added. The mixture was incubated at 4°C for 15 minutes, followed by centrifugation in 300 g for 10 minutes and collection of the precipitate conjugated with target cells (i.e. a fetal cell). Subsequently, the precipitate was suspended into the PBS buffer containing 2% FBS, and loaded onto an immunomagnetic bead sorting system (Miltenyi Biotec, Germany), followed by collection of outflow containing the target cells negative for CD45. After centrifugation of the outflow collected, the precipitate portion was suspended into the PBS buffer containing 2% FBS, thus obtaining a second solution with CD45-negative cells.

To the second solution, the CD45 antibody was added additionally. The mixture was incubated at 4°C for 15 minutes, followed by centrifugation and collection of the precipitate. Afterwards, the precipitated cells were suspended into the PBS buffer containing 2% FBS, and then incubated with 50 $\mu$L staining solution containing Hoechst 33342/PI dyes (1mg/mL) at room temperature in a dark place for 30 minutes. Following that the mixture with stained cells was filtered and transferred into a tube specialized for flow cytometer, and Hoechst positive, PI and CD45-negative cells were sorted out and collected into a PCR tube with PBS containing 1% BSA.

The product in the PCR tube was placed onto a slide, followed by picking up a nucleated single cell (i.e. the target cell) having complete cell morphology under a bright field and exhibiting desired staining results under a fluorescence field (i.e. merged "purple" under the fluorescence field, based on the selected Hoechst 33342/PI dyes) into individual wells of a 96-well plate, with 4 µL PBS per well as a base solution. Afterwards, the plate was centrifuged and sealed with a film for next whole genome amplification. In the example 1, a total of 114 single cells were picked up from the 10 mL peripheral blood sample by a mouth-controlled pipette.

**Example 2 Whole genome amplification for target cell**

The single cells obtained in the Example 1 were subjected to whole genome amplification by steps as follows.

Step 1. Preparation of a kit for whole genome amplification for the single cell

The kit contains a lysing buffer for cell denaturation, a neutralizing buffer and an amplification system.
The lysing buffer for cell denaturation consists of 0.1 mM KOH, 1 mM EDTA, 0.1 M DTT and water.
The neutralizing buffer consists of 60 mM $KH_2PO_4$, 5 mM $K_2HPO_4$ and water.
The amplification system includes $10\times$Phi29 buffer, water, 1.8 to $2.2\times10^{-8}$mol dNTPs, N8 primers (at a total concentration of 1.8 to $2.2\times10^{-11}$mol), 4.5 to $5.5\times10^{-3}$mg BSA, 0.0045 to 0.0055µL Pluronic F68 and 9 to 11 U Phi29 DNA polymerase. Specifically, the amplification system (total 40.3 µL) consists of 30 µL $H_2O$, 5µL $10\times$Phi29 buffer, 2 µL dNTPs, 2 µL N8 primers, 0.25 µL BSA (20 mg/mL, aqueous), 0.05µL 10% Pluronic F68 and 1 µL Phi29 DNA polymerase.
$10\times$Phi29 buffer is from Complete Genomics (Cat. No.: MP00012).
dNTPs are from FERMENTAS (Cat. No.: R0193), including dATP, dTTP, dCTP and dGTP (each at a concentration of 10 mM).
The N8 primers are random primers consisting of 8 dNTPs arranged randomly (i.e., dATP, dTTP, dCTP and dGTP) and are at a total concentration of 10 µM.
The 10% (in a ratio of concentration to volume) Pluronic F68 is from Sigma (Cat. No.: P5556).
The Phi29 DNA polymerase is from Complete Genomics (Cat. No.: RM00024), with a concentration of 10 U/µL.

Step 2. Preparation of whole genome amplified product

The 114 single cells were individually subjected to whole genome amplification using the kit obtained in the step 1 by steps as follows.

2.1 The single cell each was incubated with 3.7 µL PBS buffer (pH 7.5, 0.1 M) and 3 µL of the lysing buffer in the kit (for cell denaturation and genome release) at 65°C for 10 minutes, followed by addition of 3 µL of the neutralizing buffer in the kit (for stopping cell lysing and adjusting the solution to be neutral), thereby obtaining a solution as a template in a volume of 9.7 µL.
2.2 The solution as the template (9.7 µL, approximate 6 pg of DNA) obtained was incubated with the amplification system in the kit at 30°C for 3 hours, followed by incubation again at 65°C for 5 minutes, thereby obtaining the whole genome amplified product of the target cell.

The whole genome amplified product obtained may be directly used for the follow-up analysis or stored at -20°C.
Thus, it is found by the present inventors that the whole genome amplified product of the single cell obtained by the amplifying method in embodiments achieves high coverage across the whole genome and excellent homogeneity, which is suitable for identifying gene identity based on STR genotyping and analyzing the chromosomal-based copy number variation in the genome, thus benefiting the detection and analysis of the a whole genome of the fetal cell in the peripheral blood of the pregnant woman.

**Example 3 Q-PCR or STR detection of the whole genome amplified product**

Individual whole genome amplified products of the 114 single cells each were subjected to Q-PCR detection using Quantifier® Trio DNA Quantification Kit (Applied Biosystems™) on real-time qPCR instrument (ViiA 7™) according to the procedures in the specifications of the kits, with 4 out of 114 single cells exhibiting an obvious amplification signal for Y-chromosome detected for the corresponding whole genome amplified products.
The whole genome amplified products of the 4 single cells, as well as parental genomes thereof were subjected to STR detection by the present inventors using the AmpFLSTR Identifier PCR Amplification Kit and the AmpFLSTR® Yfiler PCR Amplification Kit, with results obtained and listed in Table 1 as follows:

Table 1: results of common-STR and Y-STR detection

| Cell No. | ampFLSTR Identifier[1] | | ampFLSTR® Yfiler[2] | |
|---|---|---|---|---|
| | number of covered loci/total loci number | number of mapped loci/ number of covered loci | number of covered loci/total loci number | number of mapped loci/number of covered loci |
| CFCCYY-E2 | 10/16 | 10/10 | 9/16 | 8/9 |
| CFCCYY-F2 | 14/16 | 14/14 | 15/16 | 14/15 |
| CFCCYY-M6 | 8/16 | 8/8 | 6/16 | 5/6 |
| CFCCYY-P5 | 9/16 | 9/9 | 4/16 | 4/4 |
| noted: [1]: AmpFLSTR Identifier PCR Amplification Kit enables simultaneous amplification and detection of total 16 STR loci at one time; however, normally less than 16 loci can be detected because some loci will miss during the whole genome amplification for the single cell. In this context, term "number of covered loci" indicates the number of loci successfully detected after amplification, and term "number of mapped loci" indicates the number of loci which are mapped to parental loci among the covered loci. [2]: AmpFLSTR® Yfiler PCR Amplification Kit enables simultaneous amplification and detection of total 16 STR loci at one time in Y-chromosome. Similarly and normally, less than 16 loci can be detected because some loci will miss during the whole genome amplification for the single cell. In this context, term "number of covered loci" indicates the number of loci successfully detected after amplification, and term "number of mapped loci" indicates the number of loci which are mapped to paternal loci among the covered loci. | | | | |

It can be seen from Table 1, for the 4 selected single cells each exhibiting the obvious amplification signal for Y-chromosome, the whole genome amplified products each have certain coverage, with covered loci each matched to corresponding parental STR locus. In particular, the CFCCYY-F2 cell sample achieves best results, where among the total 16 loci, 14 loci are all mapped to parental genome, and similarly 14 covered loci in the Y-chromosome are mapped to paternal genome, demonstrating the CFCCYY-F2 cell is indeed derived from the fetus of the pregnant woman.

Meantime, it is found by the present inventors that some STR loci will miss for the selected cells is mainly due to incomplete cell nucleus itself, caused by (1) apoptosis of the fetal cells in the peripheral blood of the pregnant woman, (2) damage of the target cell (i.e. the fetal cell) during isolating process, which thus makes difficult to obtain a fetal cell with a complete genome. Secondly, due to the coverage cannot reach 100% because of amplification bias in the terms of amplification, thus it is inevitable for the amplified product with some loci uncovered during amplification. In addition, it is also discovered by the present inventors that a lots of homozygous loci are presented in the whole genome amplified products, which may be resulted from (1) lots of identical loci presented in patents, thereby generating homozygotes in the genome of the fetal cell, (2) allele drop-out (ADO) occurring during the amplification for the single cell.

**Example 4 low-depth and high-throughput sequencing and analysis for the whole genome amplified product of the single cell**

Specific analysis steps are as follows:

1. The whole genome of the target cell was amplified by multiple displacement amplification (MDA) for constructing a library which was further subjected to paired-end sequencing (i.e. PE90) on the HiSeq2500 system (illumina), with sequencing data obtained.
2. The sequencing date was analyzed by aligning to the Hg19 as a reference sequence with the Soap2 software, and identifying alignment efficiency, mismatch, GC content, coverage, and the like listed in Table 2.

Table 2 statistical data from low-depth and high-throughput sequencing of whole genome MDA-amplified product

| Samples | Q30(%) | GC content (%) | Read length (bp) | Read number | data volume | Coverage (%) | Read repeat ratio |
|---|---|---|---|---|---|---|---|
| CFCCYY-E2 | 92.54 | 39.58 | 90 | 16.47M | 1.48G | 6.71% | 40.74% |
| CFCCYY-P5 | 89.89 | 41.53 | 90 | 12.56M | 1.13G | 3.02% | 60.36% |
| CFCCYY-F2 | 93.47 | 37.99 | 90 | 15.87M | 1.43G | 21.89% | 0.15% |
| CFCCYY-M6 | 92.68 | 39.87 | 90 | 15.75M | 1.42G | 8.03% | 8.50% |

It can be seen from Table 2, based on substantially same data volume, the selected 4 samples present different coverages and read repeat ratios. All of the selected 4 samples can be used for high-depth sequencing, preferably the CFCCYY-F2 cell sample with high coverage and low read repeat ratio.

**Example 5 high-depth and high-throughput sequencing and analysis for the whole genome amplified product of the single cell**

The CFCCYY-F2 cell sample were subjected to high-depth and high-throughput sequencing, with data obtained for the following analysis: (1) basic bioinformation analysis; (2) ADO correction; (3) paternity testing; and (4) disease determination.

(1) Basic information analysis

The whole genome amplified product obtained by the MDA method was subjected to library construction for PE90 sequencing on the HiSeq2500 system, with sequencing data obtained. Afterwards, the sequencing date was analyzed by aligning to the Hg19 as the reference sequence with the BWA software, and identifying alignment efficiency, mismatch, GC content, coverage, and the like. Following that, SNP and INDEL of the whole genome of the target cell were identified with the GATK software based on the obtained alignment results.

(2) ADO correction

The sequencing date after SNP calling was subjected to ADO correction by the following formula.

$$P(GC/G) = \frac{P(GC)P(G/GC)}{P(G)}$$

wherein,

P(GC/G): a probability of GC heterozygous genotype at a site where G is present;
P(G/GC): a prior probability of G presentation under a certain GC content;
P(GC): a probability of GC genotype at this site; and
P(G): a total probability, i.e, the sum of the probabilities of G presentation under all possibilities.

For example, given the GC content is 41%, the prior probability of GG genotype is $(0.41/2)^2$ (equal to 0.042025), i.e., P(G/GC) is 0.042025, all the possible prior probabilities of A, T, G, C presentations listed in Table 3 under such a GC content. The relief value P(GC) is 0.5, since GG and GC genotypes can be presented at the locus for parents.

Table 3 Statistic results of possible prior probabilities

| Genotype \ base | prior probability | A | T | G | C |
|---|---|---|---|---|---|
| AA | 0.087025 | 0.9999 | 4.1844E-05 | 2.9078E-05 | 2.9078E-05 |
| TT | 0.087025 | 4.1844E-05 | 0.9999 | 2.9078E-05 | 2.9078E-05 |
| GG | 0.042025 | 3.71069E-05 | 3.71069E-05 | 0.9999 | 2.57862E-05 |
| CC | 0.042025 | 3.71069E-05 | 3.71069E-05 | 2.57862E-05 | 0.9999 |
| AT | 0.17405 | 0.499970922 | 0.499970922 | 2.9078E-05 | 2.9078E-05 |
| AG | 0.12095 | 0.499968553 | 3.94754E-05 | 0.499964539 | 2.74321E-05 |
| AC | 0.12095 | 0.499968553 | 3.94754E-05 | 2.74321E-05 | 0.499964539 |
| TG | 0.12095 | 3.94754E-05 | 0.499968553 | 0.499964539 | 2.74321E-05 |
| TC | 0.12095 | 3.94754E-05 | 0.499968553 | 2.74321E-05 | 0.499964539 |
| GC | 0.08405 | 3.71069E-05 | 3.71069E-05 | 0.499962893 | 0.499962893 |

(3) paternity testing

For a SNP site which is different between mother and father and is homozygous for both mother and father, it can be theoretically determined that this SNP site for the fetus is heterozygous with paternal B genotype contained; at the same time the probability of B presentation in the fetal chromosome is calculated. Same method is applied for probability of paternity exclusion on 90 Asian human. The probability for the biological father and the probabilities for the 90 non-father samples are subjected Z-test. The probability of the paternal base found in the fetus is 0.516 with the Z-value of 6.32, indicating that probability of the biological father is distinguishable from those of the non-father samples and the CFCCYY-F2 cell sample is derived from the fetus.

(4) Disease anlaysis

Disease genetic linkage regions from the Clinical Genomic Database (CGD) were compared to the sequencing data of the CFCCYY-F2 cell obtained in above step (1) for coverage. After the genetic regions with coverage higher than 70% were selected for disease-related mutation sites, a total of 1046 mutation sites were detected. Subsequently, the sequencing data after GATK detection and ADO correction was subjected to noninvasive monogenic disease detection, revealing that two mutated genes presented in the CFCCYY-F2 cell derived from the fetus 1 are related to two monogenic diseases, with the former gene being a likely-pathogenic gene related to bilateral absence of the vas deferens, and the latter gene being a pathogenic gene related to severe combined immunodeficiency due to adenosine deaminase deficiency. The details for such two monogenic diseases are in Table 4.

Table 4 Detection result of monogenic disease

| Family | Disease | Mutation sites | Type |
|---|---|---|---|
| Fetus 1 | Congenital bilateral absence of the vas deferens | NM_000492.3(CFTR): c. 1312A>G (p.T438A) (heterozygous) | likely-pathogenic gene |
| Fetus 1 | severe combined immunodeficiency due to adenosine deaminase deficiency | NM_000022.2(ADA): c. 302G>A (p.R101Q) (heterozygous) | pathogenic gene |

**Industrial applicability**

With the method for obtaining the target cell from the blood sample according to embodiments of the present disclosure, the target cell is obtained in form of a single cell, which is suitable for whole genome amplification, with a whole genome amplified product useful in library construction and sequencing, thus determining whole genome sequence information of the target cell in an accurate and effective way (for example, by constructing a sequencing library and whole genome sequencing). Further, based on such whole genome sequence information of the target cell, it is possible to accurately determine fetal physical condition. That is to say, the single cell obtained by the method according to embodiments is useful in detection and analysis of the whole genome of the fetal cell in the peripheral blood of the pregnant woman.

In the specification of the present disclosure, the terms "an embodiment", "some embodiments", "an example", "a specific example", "some examples" or "a particular embodiment" and the like are intended to refer to particular features, structures, materials or characteristics described by way of example or embodiment are contained in at least one embodiment or example of the disclosure. In this specification, the schematic representation of the above terms does not necessarily refer to the same embodiment or example. Moreover, the particular features, structures, materials or characteristics described may be combined in any suitable manner in one or more embodiments or examples.

**Claims**

1. A method for isolating a target cell from a blood sample for analysis of the whole genome of the target cell, wherein the target cell is a fetal cell in form of a single cell, said method comprises steps of:

   (1) subjecting the blood sample to immunomagnetic bead sorting, thereby obtaining a first sorted product containing the target cell,
   wherein the immunomagnetic bead sorting uses beads coated with an antibody,
   wherein the antibody bound on the bead is specific for a surface antigen expressed exclusively on non-target cells, and
   wherein the target cell comprised in the first sorted product is not bound to the antibody used for the immunomagnetic bead sorting;
   (2) staining a surface antigen on the first sorted product, followed by incubation, centrifugation and washing, thereby obtaining a precipitate containing the target cell, said surface antigen being at least one selected from the group consisting of CD45, CD14, CD56, CD19 and CD20; suspending the resulting precipitate with a buffer, thereby obtaining a first solution containing the target cell; incubating the first solution containing the target cell with Hoechst 33342/Propidium Iodide (PI) dyes at room temperature in a dark place, thereby obtaining a second solution containing stained cells; and sorting the second solution containing stained cells which is diluted in advance by flow cytometer, collecting as a second sorted product cells which are Hoechst-positive, PI negative, and negative for said at least one surface antigen stained on the first sorted product; and
   (3) separating the second sorted product by mouth-controlled pipette, thereby obtaining the target cell in form of the single cell.

2. The method according to claim 1, wherein the blood sample is from a pregnant woman,
   optionally, prior to step (1), the method further comprises: removing red blood cells from the blood sample,
   optionally, contacting the blood sample with a red blood cell lysis buffer in the presence of an anticoagulant, so as to remove the red blood cells from the blood sample,
   optionally, the anticoagulant is at least one selected from the group consisting of heparin, ethylenediamine tetraacetic acid, citrate, oxalate and ACD anticoagulant,
   optionally, the antibody coated on the immunomagnetic bead is against at least one of antigens selected from CD45, CD14, CD56, CD19 and CD20,
   optionally, step (3) comprises: placing the second sorted product onto a slide, and picking up a single cell having complete cell morphology under a bright field and exhibiting desired staining results under a fluorescence field by use of dyes.

3. A device for isolating a target cell from a blood sample for analysis of the whole genome of the target cell, wherein the target cell is a fetal cell in form of a single cell, said device comprises:

   an immunomagnetic bead sorting unit, configured to subject the blood sample to immunomagnetic bead sorting by using beads coated with an antibody specific for a surface antigen expressed exclusively on non-target cells, so as to obtain a first sorted product containing the target cell, wherein the target cell comprised in the first sorted product is not bound to the antibody used for the immunomagnetic bead sorting;
   a flow cytometry sorting unit, connected to the immunomagnetic bead sorting unit and configured to perform the step (2) as defined in claim 1; and
   a mouth-controlled pipette isolating unit, connected to the flow cytometry sorting unit and configured to separate the second sorted product by mouth-controlled pipette, so as to obtain the target cell in form of the single cell.

**4.** The device according to claim 3, wherein the blood sample is from a pregnant woman;
optionally, the device further comprises a red blood cell removing unit, configured to remove red blood cells from the blood sample;
optionally, the red blood cell removing unit is configured to remove red blood cells from the blood sample by: contacting the blood sample with a red blood cell lysis buffer in the presence of an anticoagulant, so as to remove the red blood cells from the blood sample;
optionally, the anticoagulant is at least one selected from the group consisting of heparin, ethylenediamine tetraacetic acid, citrate, oxalate and ACD anticoagulant;
optionally, the mouth-controlled pipette isolating unit is configured to separate the second sorted product by mouth-controlled pipette by: placing the second sorted product onto a slide, and picking up a single cell having complete cell morphology under a bright field and exhibiting desired staining results under a fluorescence field by use of dyes.

**5.** The method according to claim 1 or 2, further comprising
lysing the target cell, thereby obtaining a lysed solution containing the whole genome of the target cell; and
amplifying the whole genome of the target cell using a polymerase.

**6.** The method according to claim 5, further comprising
identifying the gene identity of the target cell based on the whole genome amplified product,
optionally, the gene identity of the target cell is identified by subjecting the whole genome amplified product to Q-PCR or STR analysis.

**7.** An apparatus for subjecting a target cell in a blood sample to whole genome amplification, comprising:

a target cell isolating device, which is the device for isolating a target cell from a blood sample as defined in claim 3 or 4;
a lysing device, connected to the target cell isolating device and configured to lyse the target cell, so as to obtain a lysed solution containing the whole genome of the target cell; and
a whole genome amplification device, connected to the lysing device and configured to amplify the whole genome of the target cell using a polymerase.

**8.** A system for determining the gene identity of a target cell in a blood sample, comprising:

the apparatus for subjecting a target cell in a blood sample to whole genome amplification as defined in claim 7, configured to subject the target cell in the blood sample to whole genome amplification, so as to obtain a whole genome amplified product; and
a device for identifying the gene identity of the target cell, connected to the whole genome amplification device as defined in claim 7 and configured to identify the gene identity of the target cell based on the whole genome amplified product,
optionally, the device for identifying the gene identity of the target cell is configured to identify the gene identity of the target cell by subjecting the whole genome amplified product to Q-PCR or STR analysis.

**9.** The method according to claim 5, further comprising
constructing a whole genome sequencing library based on the whole genome amplified product.

**10.** The method according to claim 9, further comprising
subjecting the whole genome sequencing library to sequencing.

**11.** The method according to claim 10, further comprising
determining the whole genome sequence information of the target cell based on the sequencing result;
optionally, the blood sample is from a pregnant woman,
the target cell is a single cell derived from a fetus;
optionally, the method further comprises:

determining fetal physical condition based on the whole genome sequence information of the target cell;
optionally, the fetal physical condition comprises gender, genetic relationship, chromosomal abnormality, single nucleotide polymorphism, microdeletion and microrepeats.

**EP 3 412 769 B1**

**Patentansprüche**

1. Verfahren zur Isolierung einer Zielzelle aus einer Blutprobe zur Analyse des gesamten Genoms der Zielzelle, wobei die Zielzelle eine fötale Zelle in Form einer einzelnen Zelle ist, wobei das Verfahren die folgenden Schritte umfasst:

   (1) Unterziehen der Blutprobe einer immunomagnetischen Beadsortierung, wodurch dadurch ein erstes sortiertes Produkt erhalten wird, das die Zielzelle enthält,
   wobei die immunomagnetische Beadsortierung Beads verwendet, die mit einem Antikörper beschichtet sind,
   wobei der an das Bead gebundene Antikörper spezifisch für ein Oberflächenantigen ist, das ausschließlich auf Nicht-Zielzellen exprimiert wird, und
   wobei die im ersten sortierten Produkt enthaltene Zielzelle nicht an den für die immunomagnetische Beadsortierung verwendeten Antikörper gebunden ist;
   (2) Färben eines Oberflächenantigens auf dem ersten sortierten Produkt, gefolgt von Inkubation, Zentrifugation und Waschen, wodurch ein Präzipitat erhalten wird, das die Zielzelle enthält, wobei das Oberflächenantigen mindestens eines ist, das aus der Gruppe ausgewählt ist, die aus CD45, CD14, CD56, CD19 und CD20 besteht; Suspendieren des resultierenden Präzipitats mit einem Puffer, wodurch eine erste Lösung erhalten wird, die die Zielzelle enthält; Inkubieren der ersten Lösung, die die Zielzelle enthält, mit Hoechst 33342/Propidiumjodid (PI)-Farbstoffen bei Raumtemperatur an einem dunklen Ort, wodurch eine zweite Lösung erhalten wird, die gefärbte Zellen enthält; und Sortieren der zweiten Lösung, die gefärbte Zellen enthält, die im Voraus mittels eines Durchflußzytometers verdünnt wird, wobei als zweites sortiertes Produkt Zellen gesammelt werden, die Hoechst-positiv, PI-negativ und negativ für das mindestens eine Oberflächenantigen sind, das auf dem ersten sortierten Produkt angefärbt ist; und
   (3) Abtrennen des zweiten sortierten Produkts durch eine mundgesteuerte Pipette, wodurch die Zielzelle in Form der einzelnen Zelle erhalten wird.

2. Verfahren nach Anspruch 1, wobei die Blutprobe von einer schwangeren Frau stammt, wahlweise, vor Schritt (1), umfasst das Verfahren weiterhin: Entfernen von roten Blutkörperchen aus der Blutprobe,

   wahlweise, Inkontaktbringen der Blutprobe mit einem Erythrozyten-Lysepuffer in Gegenwart eines Antikoagulans, um die Erythrozyten aus der Blutprobe zu entfernen,
   wahlweise ist das Antikoagulans mindestens eines, ausgewählt aus der Gruppe bestehend aus Heparin, Ethylendiamintetraessigsäure, Citrat, Oxalat und ACD-Antikoagulans,
   wahlweise ist der auf dem immunmagnetischen Bead beschichtete Antikörper gegen mindestens eines der Antigene, ausgewählt aus CD45, CD14, CD56, CD19 und CD20,
   wahlweise umfasst Schritt (3): Platzieren des zweiten sortierten Produkts auf einen Objektträger und Aufnehmen einer einzelnen Zelle, die unter einem Hellfeld eine vollständige Zellmorphologie aufweist und unter einem Fluoreszenzfeld unter Verwendung von Farbstoffen die gewünschten Färbeergebnisse zeigt.

3. Vorrichtung zum Isolieren einer Zielzelle aus einer Blutprobe zur Analyse des gesamten Genoms der Zielzelle, wobei die Zielzelle eine fötale Zelle in Form einer einzelnen Zelle ist, wobei die Vorrichtung umfasst:

   eine immunomagnetische Beadsortiereinheit, die so konfiguriert ist, dass sie die Blutprobe einer immunomagnetischen Beadsortierung unterzieht, indem sie Beads verwendet, die mit einem Antikörper beschichtet sind, der für ein Oberflächenantigen spezifisch ist, das ausschließlich auf Nicht-Zielzellen exprimiert wird, um so ein erstes sortiertes Produkt zu erhalten, das die Zielzelle enthält, wobei die in dem ersten sortierten Produkt enthaltene Zielzelle nicht an den für die immunomagnetische Beadsortierung verwendeten Antikörper gebunden ist;
   eine Durchflusszytometrie-Sortiereinheit, die mit der immunmagnetischen Beadsortiereinheit verbunden und so konfiguriert ist, dass sie den Schritt (2) wie in Anspruch 1 definiert durchführt; und
   eine mundgesteuerte Pipetten-Isoliereinheit, die mit der Durchflusszytometrie-Sortiereinheit verbunden ist, und konfiguriert ist, um das zweite sortierte Produkt durch eine mundgesteuerte Pipette zu trennen, um die Zielzelle in Form der einzelnen Zelle zu erhalten.

4. Die Vorrichtung nach Anspruch 3, wobei die Blutprobe von einer schwangeren Frau stammt;

   wahlweise umfasst die Vorrichtung ferner eine Einheit zum Entfernen roter Blutkörperchen, die konfiguriert ist, um rote Blutkörperchen aus der Blutprobe zu entfernen;
   wahlweise ist die Einheit zur Entfernung roter Blutzellen so konfiguriert, dass sie rote Blutzellen aus der Blutprobe

EP 3 412 769 B1

entfernt, indem: die Blutprobe mit einem Puffer zur Lyse roter Blutzellen in Gegenwart eines Antikoagulans in Kontakt gebracht wird, um die roten Blutzellen aus der Blutprobe zu entfernen;
wahlweise ist das Antikoagulans mindestens eines, ausgewählt aus der Gruppe bestehend aus Heparin, Ethylendiamintetraessigsäure, Citrat, Oxalat und ACD-Antikoagulans;
wahlweise ist die mundgesteuerter Pipetten-Isoliereinheit konfiguriert, um das zweite sortierte Produkt durch mundgesteuerte Pipette zu trennen, indem: das zweite sortierte Produkt auf einen Objektträger gelegt wird und eine einzelne Zelle aufgenommen wird, die eine vollständige Zellmorphologie unter einem hellen Feld aufweist und gewünschte Färbeergebnisse unter einem Fluoreszenzfeld durch Verwendung von Farbstoffen zeigt.

5.  Verfahren nach Anspruch 1 oder 2, ferner umfassend

Lysieren der Zielzelle, wodurch eine lysierte Lösung erhalten wird, die das gesamte Genom der Zielzelle enthält; und
Amplifizieren des gesamten Genoms der Zielzelle unter Verwendung einer Polymerase.

6.  Verfahren nach Anspruch 5, ferner umfassend

das Identifizieren der Genidentität der Zielzelle auf der Grundlage des amplifizierten gesamten Genomprodukts, wobei wahlweise die Genidentität der Zielzelle identifiziert wird, indem das amplifizierte gesamten Genoprodukt einer Q-PCR oder STR-Analyse unterzogen wird.

7.  Vorrichtung, um eine Zielzelle in einer Blutprobe einer gesamten Genom-Amplifikation zu unterziehen, umfassend:

eine Zielzellen-Isoliervorrichtung, die die Vorrichtung zur Isolierung einer Zielzelle aus einer Blutprobe gemäß Anspruch 3 oder 4 ist;
eine Lysiervorrichtung, die mit der Zielzellen-Isoliervorrichtung verbunden und konfiguriert ist, um die Zielzelle zu lysieren, um eine lysierte Lösung zu erhalten, die das gesamte Genom der Zielzelle enthält; und
eine Gesamtgenom-Amplifikationsvorrichtung, die mit der Lysiervorrichtung verbunden und so konfiguriert ist, dass sie das gesamte Genom der Zielzelle unter Verwendung einer Polymerase amplifiziert.

8.  System zur Bestimmung der Genidentität einer Zielzelle in einer Blutprobe, umfassend:

die Vorrichtung, um eine Zielzelle in einer Blutprobe einer Gesamtgenom-Amplifikation zu unterziehen, wie in Anspruch 7 definiert, konfiguriert, um die Zielzelle in der Blutprobe einer Gesamtgenom-Amplifikation zu unterziehen, um ein amplifiziertes Gesamtgenom-Produkt zu erhalten; und
eine Vorrichtung zum Identifizieren der Genidentität der Zielzelle, die mit der Gesamtgenom-Amplifikationsvorrichtung gemäß Anspruch 7 verbunden und so konfiguriert ist, dass sie die Genidentität der Zielzelle basierend auf dem amplifizierten Gesamtgenomprodukt identifiziert,
wobei die Vorrichtung zum Identifizieren der Genidentität der Zielzelle wahlweise so konfiguriert ist, dass sie die Genidentität der Zielzelle identifiziert, indem sie das amplifizierte Gesamtgenom-Produkt einer Q-PCR oder STR-Analyse unterzieht.

9.  Verfahren nach Anspruch 5, ferner umfassend
Konstruieren einer Gesamtgenom-Sequenzierungsbibliothek basierend auf dem amplifizierten gesamten Genomprodukt.

10.  Verfahren nach Anspruch 9, ferner umfassend
Unterziehen der Gesamtgenom-Sequenzierungsbibliothek einer Sequenzierung.

11.  Verfahren nach Anspruch 10, ferner umfassend
Bestimmen der Gesamtgenom-Sequenzinformation der Zielzelle auf der Grundlage des Sequenzierergebnisses;
wahlweise ist die Blutprobe von einer schwangeren Frau, die Zielzelle ist eine einzelne Zelle, die von einem Fötus stammt;
wahlweise umfasst das Verfahren des Weiteren:

Bestimmen des fötalen physischen Zustands auf der Basis der Gesamtgenomsequenzinformation der Zielzelle;
wahlweise umfasst der fötale physikalische Zustand Geschlecht, genetische Verwandtschaft, Chromosomenanomalie, Einzelnukleotidpolymorphismus, Mikrodeletion und Mikrorepetitionen.

**Revendications**

1. Procédé d'isolation d'une cellule cible à partir d'un échantillon de sang pour analyse du génome entier de la cellule cible, sachant que la cellule cible est une cellule fœtale sous forme d'une cellule unique, ledit procédé comprenant les étapes de :

   (1) soumission de l'échantillon de sang à un tri par billes immunomagnétiques, ce qui permet d'obtenir un premier produit trié contenant la cellule cible,
   sachant que le tri par billes immunomagnétiques utilise des billes revêtues d'un anticorps, sachant que l'anticorps lié à la bille est spécifique d'un antigène de surface exprimé exclusivement sur des cellules non cibles, et sachant que la cellule cible comprise dans le premier produit trié n'est pas liée à l'anticorps utilisé pour le tri par billes immunomagnétiques ;
   (2) coloration d'un antigène de surface sur le premier produit trié, suivie par une incubation, une centrifugation et un lavage, ce qui permet d'obtenir un précipité contenant la cellule cible, ledit antigène de surface étant au moins un antigène sélectionné dans le groupe constitué par CD45, CD14, CD56, CD19 et CD20 ; suspension du précipité résultant avec un tampon, ce qui permet d'obtenir une première solution contenant la cellule cible ; incubation de la première solution contenant la cellule cible avec des colorants de Hoechst 33342/iodure de propidium (PI) à température ambiante dans un endroit sombre, ce qui permet d'obtenir une deuxième solution contenant des cellules colorées ; et tri de la deuxième solution contenant des cellules colorées qui est diluée préalablement par cytomètre en flux, collecte, comme deuxième produit trié, de cellules qui sont Hoechst-positives, PI-négatives, et négatives pour ledit au moins un antigène de surface coloré sur le premier produit trié ; et
   (3) séparation du deuxième produit trié par pipetage à la bouche, ce qui permet d'obtenir la cellule cible sous forme de la cellule unique.

2. Le procédé selon la revendication 1, sachant que l'échantillon de sang est issu d'une femme enceinte, facultativement, avant l'étape (1), le procédé comprend en outre : l'enlèvement de globules rouges de l'échantillon de sang,
   facultativement, la mise en contact de l'échantillon de sang avec un tampon de lyse de globules rouges en présence d'un anticoagulant, de manière à enlever les globules rouges de l'échantillon de sang,
   facultativement, l'anticoagulant est au moins un anticoagulant sélectionné dans le groupe constitué par l'héparine, l'acide tétraacétique d'éthylènediamine, le citrate, l'oxalate et l'anticoagulant ACD,
   facultativement, l'anticorps revêtu sur la bille immunomagnétique agit contre au moins un des antigènes sélectionnés parmi CD45, CD14, CD56, CD19 et CD20,
   facultativement, l'étape (3) comprend : le placement du deuxième produit trié sur une diapositive, et le prélèvement d'une cellule unique présentant une morphologie cellulaire complète sous un champ lumineux et l'exposition de résultats de coloration souhaités sous un champ de fluorescence moyennant des colorants.

3. Dispositif d'isolation d'une cellule cible à partir d'un échantillon de sang pour analyse du génome entier de la cellule cible, sachant que la cellule cible est une cellule fœtale sous forme d'une cellule unique, ledit dispositif comprenant :

   une unité de tri par billes immunomagnétiques, configurée pour soumettre l'échantillon de sang à un tri par billes immunomagnétiques moyennant des billes revêtues d'un anticorps spécifique d'un antigène de surface exprimé exclusivement sur des cellules non cibles, de manière à obtenir un premier produit trié contenant la cellule cible, sachant que la cellule cible comprise dans le premier produit trié n'est pas liée à l'anticorps utilisé pour le tri par billes immunomagnétiques ;
   une unité de tri par cytométrie en flux, raccordée à l'unité de tri par billes immunomagnétiques et configurée pour effectuer l'étape (2) telle que définie dans la revendication 1 ; et
   une unité d'isolation par pipetage à la bouche, raccordée à l'unité de cytométrie en flux et configurée pour séparer le deuxième produit trié par pipetage à la bouche, de manière à obtenir la cellule cible sous forme de la cellule unique.

4. Le dispositif selon la revendication 3, sachant que l'échantillon de sang est issu d'une femme enceinte ;
   facultativement, le dispositif comprend en outre une unité d'enlèvement de globules rouges, configurée pour enlever des globules rouges de l'échantillon de sang ;
   facultativement, l'unité d'enlèvement de globules rouges est configurée pour enlever des globules rouges de l'échantillon de sang par : mise en contact de l'échantillon de sang avec un tampon de lyse de globules rouges en présence d'un anticoagulant, de manière à enlever les globules rouges de l'échantillon de sang ;

facultativement, l'anticoagulant est au moins un anticoagulant sélectionné dans le groupe constitué par l'héparine, l'acide tétraacétique d'éthylènediamine, le citrate, l'oxalate et l'anticoagulant ACD ;

facultativement, l'unité d'isolation par pipetage à la bouche est configurée pour séparer le deuxième produit trié par pipetage à la bouche par : placement du deuxième produit trié sur une diapositive, et prélèvement d'une cellule unique présentant une morphologie cellulaire complète sous un champ lumineux et exposition de résultats de coloration souhaités sous un champ de fluorescence moyennant des colorants.

5. Le procédé selon la revendication 1 ou 2, comprenant en outre
la lyse de la cellule cible, ce qui permet d'obtenir une solution lysée contenant le génome entier de la cellule cible ; et
l'amplification du génome entier de la cellule cible moyennant une polymérase.

6. Le procédé selon la revendication 5, comprenant en outre
l'identification de l'identité génique de la cellule cible sur la base du produit à génome entier amplifié,
facultativement, l'identité génique de la cellule cible est identifiée par soumission du produit à génome entier amplifié à une analyse Q-PCR ou STR.

7. Appareil de soumission de cellule cible dans un échantillon de sang à une amplification de génome entier, comprenant :

un dispositif d'isolation de cellule cible, qui est le dispositif d'isolation de cellule cible à partir d'un échantillon de sang tel que défini dans la revendication 3 ou 4 ;
un dispositif de lyse, raccordé au dispositif d'isolation de cellule cible et configuré pour lyser la cellule cible, de manière à obtenir une solution lysée contenant le génome entier de la cellule cible ; et
un dispositif d'amplification de génome entier, raccordé au dispositif de lyse et configuré pour amplifier le génome entier de la cellule cible moyennant une polymérase.

8. Système de détermination de l'identité génique d'une cellule cible dans un échantillon de sang, comprenant :

l'appareil de soumission de cellule cible dans un échantillon de sang à une amplification de génome entier tel que défini dans la revendication 7, configuré pour soumettre la cellule cible dans l'échantillon de sang à une amplification de génome entier, de manière à obtenir un produit à génome entier amplifié ; et
un dispositif d'identification de l'identité génique de la cellule cible, raccordé au dispositif d'amplification de génome entier tel que défini dans la revendication 7 et configuré pour identifier l'identité génique de la cellule cible sur la base du produit à génome entier amplifié,
facultativement, le dispositif d'identification de l'identité génique de la cellule cible est configuré pour identifier l'identité génique de la cellule cible par soumission du produit à génome entier amplifié à une analyse Q-PCR ou STR.

9. Le procédé selon la revendication 5, comprenant en outre
la création d'une bibliothèque de séquençage de génome entier sur la base du produit à génome entier amplifié.

10. Le procédé selon la revendication 9, comprenant en outre
la soumission de la bibliothèque de séquençage de génome entier à un séquençage.

11. Le procédé selon la revendication 10, comprenant en outre
la détermination des informations de séquence de génome entier de la cellule cible sur la base du résultat de séquençage ;
facultativement, l'échantillon de sang est issu d'une femme enceinte,
la cellule cible est une cellule unique dérivée d'un fœtus ;
facultativement, le procédé comprend en outre :

la détermination d'un état physique fœtal sur la base des informations de séquence de génome entier de la cellule cible ;
facultativement, l'état physique fœtal comprend le genre, la relation génétique, une anomalie chromosomique, un polymorphisme nucléotidique unique, une microdélétion et des microrépétitions.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015175562 A
- US 2014228226 A

**Non-patent literature cited in the description**

- Molecular Cloning: A Laboratory Manual